# EUROPEAN PATENT APPLICATION

(11) **EP 1 604 996 A2**
(43) Date of publication of application: **14.12.2005**
(21) Application number: 05251415.5
(22) Date of filing: 09.03.2005
(51) Int. Cl.: C07K 14/415, C07K 16/16, A61K 39/35, G01N 33/53

(54) **An allergenic protein complex of natural rubber latex**

(30) Priority: 18.03.2004 MY 0400947
(71) Applicant: Malaysian Rubber Board, Kuala Lumpur 50450 (MY)
(72) Inventor: Sunderasan, Elumalai/Malaysian Rubber Board, 50450 Kuala Lumpur (MY); Arif, Siti Arija Mad/Malaysian Rubber Board, 50450 Kuala Lumpur (MY); Yeang, Hoong Yeet/Malaysian Rubber Board, 50450 Kuala Lumpur (MY)
(74) Representative: Gaunt, Robert John

(57) **Abstract**

The invention relates to the allergenic latex protein Hev b 4. The full cDNA sequence of the smaller protein components of Hev b 4 is disclosed, together with the amino acid sequence of those components and the synthesis of a recombinant protein using the aforesaid DNA sequence or a synthetic peptide derived from the amino acid sequence. The use of the Hev b 4 protein complex and of an antibody against that protein is described in immunoassays and in immunotherapy for the relief of allergy or allergy symptoms.

### Annex to the application documents - subsequently filed sequences listing

SEQUENCE LISTING

<110>  Malaysian Rubber Board

<120>  AN ALLERGENIC PROTEIN COMPLEX OF NATURAL RUBBER LATEX

<130>  RJG/JLB/4943-EP

<140>  05251415.5
<141>  2005-03-09

<150>  PI20040947
<151>  2004-03-18

<160>  4

<170>  PatentIn version 3.3

<210>  1
<211>  1375
<212>  DNA
<213>  Hevea brasiliensis

<400>  1
accacaaaga caaagaacct gcacaagcta aatccatata gtgcaatggc aagtctagct      60

tattccttgt tcattttaag cctatttacc ttcaccctgc tcaacccagt ttgtaccgag     120

ctggatgaat atttgtttag ctttggggat ggactttatg atgcaggcaa tgctaaattt     180

atatatcctg ataagtatct tccctcttat caccatccat atggtaccac tttcttcgac     240

tatcctacag gaagattttc tgatggtcgc acagttgttg attttgttgc tgaaaatgtg     300

agcttacctc gtattccacc gttcaaaaac aaggaagcaa atttcactta tggcgccaat     360

ttcgcttcag aaggtgcaac tgcttcggat agtaatccgc taatcgactt tcgaagccag     420

attagggact tcggagagtt gaaactggaa tgggcagtcc aactagttaa tgtaaccgaa     480

ctagcaagga ggctgaaaaa ggcagtttac ttaatttcct ttggggctga tgattatttg     540

aattatgaaa taccttctga agccagccga gaacaattgg aaagtattgt ggatgtagtg     600

cttggcaaca tttcggacag gatcaaggaa ttgtatgatt ttggagcaag gaaatttgta     660

gttgagaacg ttgcacccct aggcttaatt ccatttatta aacaaacgtc cgacaattct     720

acactatttt atgaacttgc atcattgcac gcaatgaaac tacctcaaat cctcgagaaa     780

atacaagatg ggtatctttt tccagaattc aactatacag tcttcaatta cttcggcatc     840

ataaaagaaa ttatagatgc acctggcgaa catggcttca agtacgggga cattgcatgc     900

tgtggcaatt cgacatatcg tgggcaagct tgtggatttt tggattatga attttgtgtg 960

tgtggtaata agactgagta tttgtttttt gacggtaccc ataacactga tgccgccaat 1020

aatctactag ctgaattaat gtgggacaag gaatcaggat tcatatcacc ttatggcgtt 1080

aaggacttct ttccatctcc tactaccatt cagacccttc ttaccgaagc tactgcatta 1140

ggttaattga aggcatatat attatttaag ttactgtgtc tgctatatat atgtgtctgt 1200

atttctaata attaaataag gcaacaaatt cgatatagtt tttgttgtcc ctactaagga 1260

gttcagaagg atggatggat tgaactcttt tacgtggttc agctattgcg tgtagctagc 1320

ctattggaaa atctcatcta tgccaaattc agtaaaaaaa aaaaaaaaaa aaaaa 1375

<210> 2
<211> 366
<212> PRT
<213> Hevea brasiliensis

<400> 2

Met Ala Ser Leu Ala Tyr Ser Leu Phe Ile Leu Ser Leu Phe Thr Phe
1               5                   10                  15

Thr Leu Leu Asn Pro Val Cys Thr Glu Leu Asp Glu Tyr Leu Phe Ser
            20                  25                  30

Phe Gly Asp Gly Leu Tyr Asp Ala Gly Asn Ala Lys Phe Ile Tyr Pro
        35                  40                  45

Asp Lys Tyr Leu Pro Ser Tyr His His Pro Tyr Gly Thr Thr Phe Phe
    50                  55                  60

Asp Tyr Pro Thr Gly Arg Phe Ser Asp Gly Arg Thr Val Val Asp Phe
65                  70                  75                  80

Val Ala Glu Asn Val Ser Leu Pro Arg Ile Pro Pro Phe Lys Asn Lys
                85                  90                  95

Glu Ala Asn Phe Thr Tyr Gly Ala Asn Phe Ala Ser Glu Gly Ala Thr
            100                 105                 110

Ala Ser Asp Ser Asn Pro Leu Ile Asp Phe Arg Ser Gln Ile Arg Asp

115                 120                 125

Phe Gly Glu Leu Lys Leu Glu Trp Ala Val Gln Leu Val Asn Val Thr
    130                 135                 140

Glu Leu Ala Arg Arg Leu Lys Lys Ala Val Tyr Leu Ile Ser Phe Gly
145                 150                 155                 160

Ala Asp Asp Tyr Leu Asn Tyr Glu Ile Pro Ser Glu Ala Ser Arg Glu
                165                 170                 175

Gln Leu Glu Ser Ile Val Asp Val Val Leu Gly Asn Ile Ser Asp Arg
            180                 185                 190

Ile Lys Glu Leu Tyr Asp Phe Gly Ala Arg Lys Phe Val Val Glu Asn
        195                 200                 205

Val Ala Pro Leu Gly Leu Ile Pro Phe Ile Lys Gln Thr Ser Asp Asn
    210                 215                 220

Ser Thr Leu Phe Tyr Glu Leu Ala Ser Leu His Ala Met Lys Leu Pro
225                 230                 235                 240

Gln Ile Leu Glu Lys Ile Gln Asp Gly Tyr Leu Phe Pro Glu Phe Asn
                245                 250                 255

Tyr Thr Val Phe Asn Tyr Phe Gly Ile Ile Lys Glu Ile Ile Asp Ala
            260                 265                 270

Pro Gly Glu His Gly Phe Lys Tyr Gly Asp Ile Ala Cys Cys Gly Asn
        275                 280                 285

Ser Thr Tyr Arg Gly Gln Ala Cys Gly Phe Leu Asp Tyr Glu Phe Cys
    290                 295                 300

Val Cys Gly Asn Lys Thr Glu Tyr Leu Phe Phe Asp Gly Thr His Asn
305                 310                 315                 320

Thr Asp Ala Ala Asn Asn Leu Leu Ala Glu Leu Met Trp Asp Lys Glu
                325                 330                 335

Ser Gly Phe Ile Ser Pro Tyr Gly Val Lys Asp Phe Phe Pro Ser Pro
            340                 345                 350

Thr Thr Ile Gln Thr Leu Leu Thr Glu Ala Thr Ala Leu Gly
        355                 360                 365

<210>  3
<211>  40
<212>  PRT
<213>  Hevea brasiliensis

<400>  3

Glu Leu Asp Glu Tyr Leu Phe Ser Phe Gly Asp Gly Leu Tyr Asp Ala
1               5                   10                  15

Gly Asn Ala Lys Phe Ile Tyr Pro Asp Lys Tyr Leu Pro Ser Tyr His
            20                  25                  30

His Pro Tyr Gly Thr Thr Phe Phe
        35                  40

<210>  4
<211>  120
<212>  DNA
<213>  Hevea brasiliensis

<400>  4
gagctggatg aatatttgtt tagctttggg gatggacttt atgatgcagg caatgctaaa      60

tttatatatc ctgataagta tcttccctct tatcaccatc catatggtac cactttcttc     120

## Description

This invention relates to the allergenic latex protein Hev b 4. More particularly it relates to the full cDNA sequence of the smaller protein components of Hev b 4, the amino acid sequence of those components and the use of the Hev b 4 protein complex in immunoassays and in immunotherapy.

### Background of the invention

Proteins constitute about 1-2% fresh weight of *Hevea brasiliensis* latex. About 70% of latex proteins are found in the aqueous phase, with the remainder being associated or bound to organelles including rubber particles. A small proportion of these latex proteins are known to be allergenic in that they can induce an allergic response in sensitised persons (Yeang *et al*., 2002). At present, there are thirteen allergenic latex proteins recognised by the International Union of Immunological Societies (IUIS). These are named Hev b 1 to Hev b 13 according to the IUIS-World Health Organisation nomenclature for allergens.

In the original patent application on latex allergens filed in Malaysia (PI 9402468, 16 September 1994) and subsequently in other countries (e.g. EP-A-0704457), three latex allergens were claimed. These were *Hev b* II, *Hev b* III and *Hev b* IV, which, under the revised IUIS nomenclature, are presently known as Hev b 2, Hev b 3 and Hev b 4 respectively. In the patent specification, Hev b 4 was described as being a component of the latex microhelix complex located in a latex organelle known as the lutoid. Hence, Hev b 4 is a protein complex, rather than a single protein. When the protein complex is subjected to SDS-polyacrylamide gel electrophoresis (SDS-PAGE), it separates into three components, with the largest protein discerned at 56 kDa followed by two smaller proteins of about 50 kDa. The allergenicity of all three components of the protein complex was demonstrated by their ability to bind IgE antibody of latex allergic patients' sera (Sunderasan *et al*., 1995). The N-terminal sequence of one of the Hev b 4 components appeared in the aforementioned Malaysian patent specification, but the sequence did not match any of the known proteins in the databases at the time. The complete amino acid sequence of any of the three proteins constituting Hev b 4, and the cDNA sequences encoding these proteins were not available at the time the patent was filed. In subsequent research, it was shown that the larger protein component was a cyanogenic β-glucosidase (Sunderasan *et al*., 2002). The smaller protein components (which later research would show to be the main allergenic components of Hev b 4) remained unelucidated.

### Summary of the invention

In the current patent application, the full cDNA sequence of the smaller protein components (approx. 50 kDa) of Hev b 4 is presented. From the DNA sequence, the complete amino acid sequence of the smaller protein components of Hev b 4 is deduced. Evidence is presented to show that although both the larger and smaller components of Hev b 4 are allergenic, prevalence of sensitisation to the smaller components is higher as compared with the larger component (β-glucosidase).

### Brief Description of the Figures

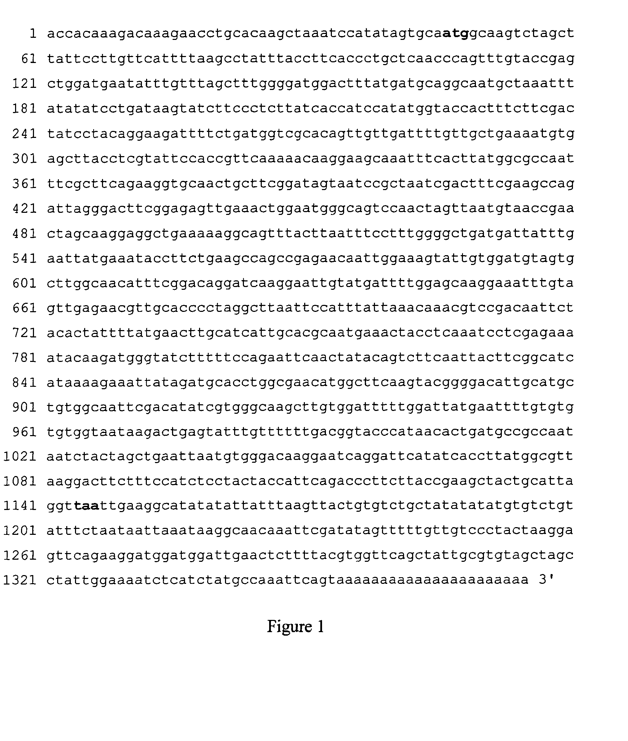

Figure 1 shows the DNA sequence (1375 bp) of the full-length cDNA clone encoding the smaller components of the *Hevea brasiliensis* latex allergenic protein complex, Hev b 4, where a, g, c and t are the abbreviations for the nucleotide bases adenine, guanine, cytosine and thymine respectively. The potential translation start codon (ATG) and the stop codon (TAA) of the predicted open reading frame are marked in bold.

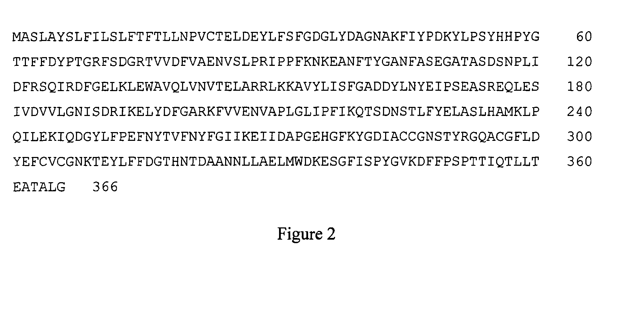

Figure 2 shows the amino acid sequence of the smaller components of Hev b 4 derived from the translation of the corresponding cDNA as shown in Figure 1. The letters of the alphabet are accepted codes for the amino acids (Cohn, 1984).

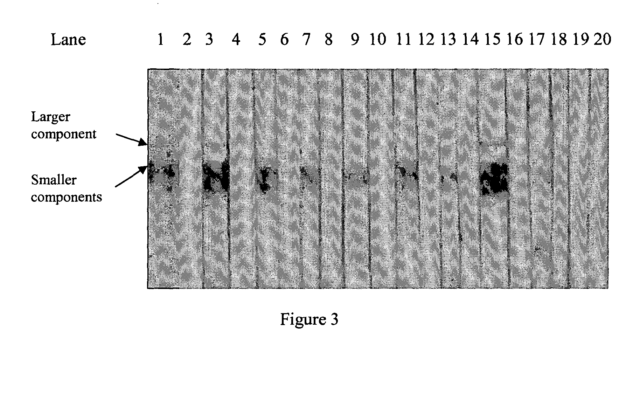

Figure 3 shows Hev b 4 protein complex and its Western blot after separation by SDS-polyacrylamide gel electrophoresis. The protein is stained with Coomassie Blue to show the presence and electrophoretic migration of Hev b 4 (lane 1). Normal human sera showing no IgE binding to Hev b 4 blot (lane 2) serves as a negative control. Binding of human IgE of patients sensitised to Hev b 4 on the Western blot (lanes 3, 5, 7, 9, 11, 13, 15 and 17). Only two patients (lanes 3 and 15) from the eight positive to Hev b 4 show strong IgE binding to the larger component of Hev b 4. Latex allergic patients' sera not reacting to Hev b 4 (Lanes 4, 6, 8, 10, 12, 14, 16, 18, 19 and 20).

### Detailed description of the invention

The present invention relates to a cDNA sequence of the smaller components of Hev b 4, a protein complex isolated from the B-serum of centrifuged latex obtained by tapping the rubber tree, *H. brasiliensis*. The following description also details how the N-terminal protein sequence information of the smaller components can be used in cloning the corresponding cDNA, and how the protein can be used in immunoassay and immunotherapy.

### Amino acid sequencing of the protein

The two protein bands comprising the smaller components of Hev b 4 were excised following SDS-PAGE. N-terminal sequencing performed on the first protein band of the smaller components of Hev b 4 yielded a stretch of forty amino acid residues (shown below); an extension of twenty residues compared to the sequence submitted in the original patent application were obtained. N-terminal sequencing of the second band of the smaller components of Hev b 4 was terminated after the seventh residue, as they appeared identical to that of the first band. The single alphabet representations are accepted abbreviations for individual amino acids.
N-terminal sequence: ELDEYLFSFG DGLYDAGNAK FIYPDKYLPS YHHPYGTTFF

The amino acid sequence data is compared against the protein sequence database of the National Centre for Biotechnology Information (NCBI), USA, using the Basic Local Alignment Search Tool (BLAST) algorithm. The search revealed that the query sequences had good homology to plant GDSL-like lipases and myrosinase-associated proteins of crucifers.

### Preparation and cloning of the cDNA encoding the smaller components of Hev b4

The complementary DNA (cDNA) encoding the amino acid sequence of the smaller components of Hev b 4 can be cloned and multiplied in a host such as a micro-organism. The micro-organism can be selected from the group consisting of bacteria, yeast, and viruses. Eukaryotic cells can also be used as vectors. The following is a description of the method used to clone the cDNA of the smaller components of Hev b 4. Standard procedures are used in the preparation and purification of DNA, agarose gel electrophoresis, ligations, transformations and total latex RNA isolation.

### Preparation of latex total RNA

Latex is collected by tapping the *H. brasiliensis* tree. Before the tree is tapped, it is fitted with a sterilised drainage spout. Immediately upon tapping, the incision and spout are washed with about 20 ml of 2X RNA extraction buffer (0.1 M Tris-HCl, 0.3 M LiCl, 0.01 M EDTA, 10% SDS, pH 9.5). The latex is then washed down with 100 ml of 2X RNA extraction buffer to a total collected volume of 200 ml in a sterile conical flask. In the laboratory, the latex is mixed well and centrifuged in polyallomer tubes at 40,000 g for 30 minutes at 15°C. The aqueous phase is gently decanted into sterile centrifuge tubes and subsequent processing of the aqueous phase to isolate total RNA is performed according to the method of Prescott and Martin (1987).

### Synthesis of the cDNA encoding the smaller components of Hev b 4

First strand cDNA synthesis is prepared by reverse transcribing 2.5 µg of total RNA in a 25 µl reaction mixture using the 5'/3' RACE™ Kit (Invitrogen, USA) as per vendor's instructions. Synthesis of double stranded cDNA is accomplished by PCR amplification.

The complete cDNA sequence of the smaller components of Hev b 4 was obtained by combining both their 5' and 3' RACE products. An antisense degenerate primer designed based on conserved regions within plant GDSL-like lipases is used to initiate the first strand synthesis to capture the 5' terminus. Oligo-dT primer is used to reverse transcribe the 3' terminus of the transcript. An aliquot of the reverse transcription containing the 5' terminus of the transcript is amplified in a thermocycler using the combination of the abridged anchor primer (supplied by the manufacturer) and antisense degenerate gene specific primer. Similarly, an aliquot of the reverse transcription containing the 3' terminus of the transcript is amplified using the combination of oligo-dT and sense-strand gene specific primer. A second round of PCR is performed using 2 µl of the first round amplifications as the template. 10 µl of each secondary PCR is used for analysis on a 1.2% agarose gel containing ethidium bromide for detection of DNA.

### Cloning of the cDNA encoding the smaller components of Hev b 4

The 5' and 3' RACE products are ligated into pGEM-T® vector (Promega, USA) as per the vendor's instructions. The ligates (3 µl) are used for the transformation of the vector into *Escherichia coli* JM109 (Promega, USA). After incubating overnight at 37°C in LB agar medium containing amplicillin (100 µg/ml) and Xgal (80 µg/ml)/IPTG (3 mmol/L), putative transformants that appear as white colonies were selected from the background of blue colonies. The picked clones are screened using the Wizard® SV Minipreps DNA Purification System (Promega, USA) as per vendor's instructions. One µg plasmid DNA of the selected clones were then sent for nucleic acid sequencing.

The 5' and 3' DNA sequences were combined and translated into amino acids that they encoded (see Figures 1 and 2). The full sequence was elucidated for the cloned cDNA of 1375 base pairs. The predicted protein contained 366 amino acid residues that included a signal peptide of 24 amino acids. The calculated molecular weight of the mature protein is 38529.2 Da. There is a discrepancy between this molecular size (38.5 kDa) and the observed molecular size as indicated by SDS-polyacrylamide gel electrophresis (approximately 50 kDa). This difference is due to the carbohydrate component of the protein. The presence of the carbohydrate was confirmed using the GlycoTrack carbohydrate detection kit (Oxford Glycosystems, United Kingdom) following the manufacturer's instructions.

A search in the protein domain database using the deduced amino acid sequences revealed the presence of lecithinase conserved domains. The deduced amino acid sequence data also encompassed the N-terminal segment obtained independently from protein sequencing.
gagctggatgaatatttgtttagctttggggatggactttatgatgcaggcaatgctaaatttatatatcctgataagtatctt ccctctatcaccatccatatggtaccactttcttc (at position 118 to 237 of the cDNA) encodes the peptide ELDEYLFSFG DGLYDAGNAK FIYPDKYLPS YHHPYGTTFF
where a,g,c and t are the abbreviations for the nucleotide bases adenine, guanine, cytosine and thymine respectively. The applicants note that minor variations of the DNA sequence would not alter substantially the basic characteristics of the peptide that the DNA encodes.

### Demonstration of protein allergenicity

A Western blot of the purified Hev b 4 protein complex was incubated with blood serum from latex allergic patients to determine if IgE (the immunoglobulin that mediates the allergic reaction) binds to the protein. Binding of IgE to the protein indicated that the protein is allergenic.

Hev b 4 protein complex is separated by SDS-polyacrylamide gel electrophoresis (SDS-PAGE) on 12.5% gels and transferred electrophoretically to a nitrocellulose membrane to obtain a Western Blot. The membrane is then pre-incubated with PBS containing 5% non-fat milk. To detect protein-IgE binding in Western blots the nitrocellulose membrane is incubated with serum of several latex-allergic patients (diluted 1:5.25 in PBS-milk and 0.05% sodium azide) as the primary antibody. Anti-human IgE conjugated to alkaline phosphatase served as the secondary antibody. The binding of human IgE to Hev b 4 on a Western blot of the protein after separation of the protein by SDS-polyacrylamide gel electrophoresis is shown in Figure 3.

### Use of the allergenic protein complex, Hev b 4 in immunoassays

Native or recombinant smaller components of Hev b 4 or its molecular variant (a protein similar to the smaller components of Hev b 4, but differing slightly, for example, in a few amino acids) can be used on its own, or in combination with an antibody developed against the smaller components of Hev b 4 or its molecular variant, in immunoassays. Molecular variants of the smaller components of Hev b 4 may occur naturally in latex or may exist as a result of laboratory manipulation. Such immunoassays can be constructed in many different formats, but they rely on the immunological reaction between an antibody and its antigen. The antibody in this instance can be human IgE or an antibody against the smaller components of Hev b 4, or its molecular variant. Its antigen can be native or recombinant smaller components of Hev b 4 or its molecular variant, or a peptide that embodies the epitope site of the protein or its molecular variant.
Immunoassay can be used for the diagnosis of allergy to the smaller components of Hev b 4 or allergy to latex in general. In a different format, the immunoassay can be used for the detection of the smaller components of Hev b 4 in latex or latex products.

### Use of the smaller components of Hev b 4 in immunotherapy

Immunotherapy is a preventive treatment for allergic reactions that is carried out by giving gradually increasing doses of the allergen to which the person is allergic. The incremental increases of the allergen cause the immune system to become less sensitive to the substance when the substance is encountered in the future. There are several treatment protocols for immunotherapy. As an example, immunotherapy with the smaller components of Hev b 4 can be carried out by injecting the purified sample into skin of the arm. An injection may be given once a week for about 30 weeks, after which injections can be administered every two weeks. Eventually, injections can be given every four weeks. The duration of therapy may be three or four years, sometimes longer. In place of native smaller components of Hev b 4, immunotherapy may also be carried out with a suitable recombinant smaller components of Hev b 4, but differing slightly, for example, in a few amino acids), or a peptide representing a portion of the smaller components of Hev b 4 or its molecular variant.

From the above description, one skilled in the art can easily ascertain the essential characteristics of the present invention, and without departing from the spirit and scope thereof, can make various changes and modifications of the invention to adapt it to various usage and conditions.

### References:

1. PRESCOTT, A AND MARTIN, C (1987). A rapid method for the quantitative assessment of levels of specific mRNAs in plants. Plant Mol. Biol. Reporter, 4, 219-224
2. SUNDERASAN, E., SAMSIDAR HAMZAH, SHARIFAH HAMID, WARD, M.A., YEANG, H.Y. AND CARDOSA, M.J. (1995) Latex B-Serum β-1,3-Glucanase (Hev b II) and a Component of the Microhelix (Hev b IV) are Major Latex allergens. *J*. *nat. Rubb. Res.,* **10**(2), 82-99
3. YEANG H.Y., SITI ARIJA M. ARIF, FARIDAH YUSOF, AND SUNDERASAN E. (2002). Allergenic proteins of natural rubber latex. *Methods* 27, 32-45
4. SUNDERASAN E., WARD M.A., AND YEANG H.Y. (2002). Isolation and characterisation of *Hevea brasiliensis* latex cyanogenic glucosidase. *J*. *Rubb.Res.* 5(4), 244-252
5. COHN, W.E. (1984) Nomenclature and symbolism of alpha-amino acids. *Methods Enzymo.* 106, 3-17.

## Claims

1. A protein that is one or both of the smaller components of the Hev b 4 protein complex in a substantially purified form, or a molecular variant of that protein, and that can induce allergic reaction in persons sensitized to the protein.

2. A protein as claimed in claim 1, that is a homologue of lecithinases.

3. A protein with the amino acid sequence as given in Figure 2, or portions thereof, or with minor variations in the amino acid sequence that do not result in the allergenic properties of the protein being substantially altered.

4. A protein as claimed in claim 3, with different carbohydrates bound to it or without carbohydrates bound to it.

5. A cDNA sequence encoding the protein or a portion of the protein as claimed in either claim 1 or 2, wherein the DNA sequence is as in Figure 1 or with minor variations in the sequence that do not result in the allergenic properties of the protein being substantially altered.

6. A recombinant protein synthesized using the DNA sequence in claim 5 or a synthetic peptide derived from the amino acid sequence in claim 3.

7. An antibody against the protein of any one of claims 1, 2, 3, 4 and 6.

8. The use of a native protein or its molecular variant as claimed in any one of claims 1 to 4 in immunoassays and immunotherapy.

9. The use of an antibody as claimed in claim 7 in immunoassays and immunotherapy.

10. The use of a recombinant protein or its molecular variant in recombinant form as claimed in claim 6 in immunoassays and immunotherapy for the relief of allergy or allergy symptoms.
